# NEUE EUROPÄISCHE PATENTSCHRIFT

(11) **EP 1 021 967 B2**
(45) Veröffentlichungstag und Bekanntmachung des Hinweises auf die Entscheidung über den Einspruch: **29.11.2006**
(45) Hinweis auf die Patenterteilung: 25.08.2004
(21) Anmeldenummer: 00100272.4
(22) Anmeldetag: 19.01.2000
(51) Int. Cl.: A43B 17/02

(54) **Schuheinlagensystem**
Sole insert sytem
Semelle à insérer

(30) Priorität: 20.01.1999 DE 19902147; 15.09.1999 DE 19944263
(43) Veröffentlichungstag der Anmeldung: 26.07.2000
(73) Patentinhaber: Spannrit Schuhkomponenten GmbH, 63801 Kleinostheim (DE)
(72) Erfinder: Katzer, Roland, 63801 Kleinostheim (DE)
(74) Vertreter: Leske, Thomas

(56) Entgegenhaltungen:
- AT-A- 136 121
- DE-A- 3 114 534
- DE-A- 3 506 809
- DE-A- 10 056 908
- DE-A- 19 706 808
- DE-T- 3 855 432
- DE-U- 7 622 369
- DE-U- 29 703 056
- DE-U- 29 810 518
- GB-A- 312 124
- GB-A- 943 674
- US-A- 2 409 594
- US-A- 3 265 071
- US-A- 4 813 157
- US-A- 5 438 768
- Saechtling Kunststoff Taschenbuch, Karl Oberbach, 28 Ausgabe,2001

## Beschreibung

Die vorliegende Erfindung betrifft eine individuell anpassbare Schuheinlage und ein als Halbfertigprodukt ausgebildetes Schuheinlagensystem zur Herstellung von individuell anpaßbaren orthopädischen Schuheinlagen.

Orthopädische Schuheinlagen werden bisher so gefertigt, daß einzelne vorgefertigte Schuheinlagenelemente zumindest teilweise durch Verkleben mit einem flüssigen Kontaktklebstoff in gewünschter Anordnung miteinander unlösbar verbunden werden. Dies erlaubt eine einmalige individuelle Anpassung der Schuheinlagenelemente an den Fuß des Patienten. Diese Anpassung erfolgt üblicherweise durch einen Orthopädie-Schuhmacher.

Orthopädische Schuheinlagen bestehen üblicherweise aus folgenden Schuheinlagenelementen: einem anatomisch geformten Rohling, welcher mit einem oder mehreren Lederdeckelementen versehen ist und gegebenenfalls Pelotten, welche zwischen dem Rohling und den Deckelementen eingefügt sind. Pelotten sind beispielsweise Mittelfußpoister. Die Lederdeckelemente und die Anordnung der Pelotten können individuell an den Fuß des Patienten oder Trägers angepaßt werden.

Im Stand der Technik ist ein vorgefertigtes Schuheinilagensystem bekannt, welches aus einem anatomisch geformten Rohling und aus einer Lederober- und einer Lederunterdecke besteht, wobei der Rohling zwischen diesen beiden Lederdecken angeordnet ist. Die Lederunterdecke ist hierbei ganz mit dem Rohling und mit einem Teil der Lederoberdecke verklebt. Die Lederoberdecke ist mit dem Rohling nicht verklebt. In den Raum zwischen dem Rohling und der Lederoberdecke kann der Orthopädie-Schuhmacher Schuheinlagenelemente wie Pelotten zur individuellen Anpassung an den Fuß eines Patienten einsetzen.

Nachteil dieses Schuheinlagensystems ist jedoch, daß ein flüssiger Kontaktklebstoff eingesetzt werden muß, um die Pelotten und die Lederoberdecke mit dem Rohling zu verbinden. Der Kontaktklebstoff wird auf die Schuheinlagenelemente aufgetragen und muß erst antrocknen, bevor diese miteinander verbunden werden können. Dies ist sehr zeitaufwendig und aufgrund der während des Antrocknungsprozesses des Klebstoffs entweichenden Lösungsmittel gesundheitsgefährdend mit einer erheblichen gesundheitlichen Belastung für die Person, welche die Schuheinlage verklebt.

Zwischen dem Rohling und der Lederunterdecke können keine weiteren Schuheinlagenelemente eingefügt werden, da der Rohling mit der Lederunterdecke fest verklebt ist. Durch Fußschweiß und Wärme können die Klebstoffe außerdem hart werden. Sie verlieren ihre Elastizität, was den Tragekomfort der Schuheinlagen einschränkt.

Eine einschlägige Schuheinlage ist aus DE-A-3 506 809 bekannt. Bekannt ist auch (DE 3114534 A1), zum Verkleben einen Wärmeaktivierbaren, wielderholt lös- und verbindbaren Kleber zu verwenden.

Aufgabe der vorliegenden Erfindung ist es, eine Schuheinlage zu schaffen, welche eine kostengünstige und zeitsparende Herstellung von orthopädischen Schuheinlagen, insbesondere seitens des Orthopädie-Schuhmachers erlaubt.

Diese Aufgabe wird gelöst durch eine Schuheinlage mit den Merkmalen gemäß Anspruch 1, und durch ein Schuheinlagensystem mit den Merkmalen gemäß Anspruch 4.

Demgemäß wird eine individuell anpaßbare, insbesondere orthopädische Schuheinlage bereitgestellt, welche zumindest drei verbindbare Schuheinlagenelemente, welche aus einem anatomisch geformten Rohling, zumindest einem Deckelement und zumindest einer zwischen dem Deckelement und dem Rohling angeordneten Pelotte bestehen, aufweist. Zumindest eines der Schuheinlagenelemente weist zumindest im Bereich der Pelotte ein Klebemittel auf. Erfindungsgemäß sind die Schuheinlagenelemente lösbar verbunden. Die Schuhsohle weist zusätzlich Unterdecke auf, wobei sich der Rohling nicht über die gesamte Fußfläche sondern von der Ferse bis zum Beginn des faßballens, d.h. über etwa 2/3 der Fußlänge erstreckt, die Oberdecke nich über die gesamte Fußfläche erstreckt und Ober- und Unterdecke größer ausgebildet sind als der Rohling, wobei in verklebten zustand ein nicht durch den Rolling gestützter Randbereich ausgebildet ist.

Vorzugsweise ist das Klebemittel ein wiedererweichbarer Kleber, insbesondere ein Heißkleber. Es kann ebenfalls Kautschukkleber verwendet werden. Beide Klebemittel weisen den Vorteil auf, beispielsweise nach dem Auftragen bzw. Beschichten des Rohlings und dem Ankleben einer Pelotte auf dem Rohling, unter Wärmeeinwirkung wiedererweichbar zu sein. Alle zuvor verklebten Teile sind dadurch wieder voneinander ablösbar. Zur Schaffung einer Schuheinlage wird der Überzug von dem Klebemittel auf der Oberfläche des Rohlings abgezogen. Nachdem das Klebemittel einer Wärmebehandlung unterzogen wurde, können dann Schuheinlagenelemente wie Pelotten und Deckelemente, beispielsweise Lederoberdecke und/oder Lederunterdecke aufgebracht werden. Die Schuheinlagenelemente werden auf das Klebemittel aufgelegt und nur kurz angedrückt. Die Schuheinlage ist dann sofort tragfähig. Ein Auftragen von Klebemittel entfällt, so daß auch keine gesundheitsgefährenden Lösungsmittel entweichen.

In einem Ausführungsbeispiel kann Klebemittel eine dünne, beidseitig Klebende Selbstklebefolie verwendet werden.

Bei allen hierbei verwendeten Klebemitteln ist es vorteilhaft, daß der Prozeß des Verklebens und Lösens mehrfach wiederholbar ist, d.h. die Schuheinlagenelemente der fertig verklebten Schuheinlage können zu Anprobezwecken mehrfach voneinander gelöst, neu angeordnet und wieder fest verbunden werden. Selbst nach dem Tragen einer fertig angepaßten Schuheinlage kann, wenn sich beispielsweise die Anatomie des Fußes im Laufe der Zeit leicht verändert, die bereits getragene Einlage wieder neu angepaßt werden.

Typischerweise wird die Wärmebehandlung bei Temperaturen im Bereich von 75 °C bis 150 °C durchgeführt. Oberhalb von 75 °C beginnt die Erweichung des Klebemittels, so daß Elemente, die damit beschichtet wurden, wieder voneinander lösbar sind. Da beispielsweise der Rohling des Schuheinlagensystems vorzugsweise aus Kunststoff hergestellt ist, sollte vorzugsweise eine Temperatur von etwa 150 °C bei der Wärmebehandlung nicht überschritten werden.

Vorteilhafterweise können diese Temperaturen in einem herkömmlichen Heizofen erreicht werden; es ist aber auch möglich, die Wärmebehandlung beispielsweise unter Verwendung einer Heizlampe durchzuführen.

Gemäß einem weiteren Aspekt der Erfindung weist ein als Halbfertigprodukt ausgebildetes Schuheinlagensystem mindestens zwei zu verbindende Schuheinlagenelemente. In Form eines anatomisch geformten Rohlings und zumindest eines Deckelementes auf. Zumindest eines der zu verbindenden Schuheinlagenelemente weist dabei ein Klebemittel auf, mittels welchem die Schuheinlagenelemente tragfertig verbindbar sind.

Das Klebemittel ist ein thermisch wiedererwelchbarer Kleber, insbesondere ein Heißkleber (hot melt). Gemäß einem weiteren bevorzugten Ausführungsbeispiel kann als Klebemittel auch ein wiedererweichbarer Kautschukkleber verwendet werden. Das wiedererweichbare Klebemittel hat den Vorteil, daß die Schuheinlagenelemente eine zur Verarbeitung des Schuheinlagensystems zu einer Individuell angepaßten Schuheinlage fertige Klebemittelbeschichtung bieten, die durch einfaches Erwärmen weiterverarbeitbar ist.

In einem Ausführungsbeispiel ist als Klebemittel eine dünne, beidseitig klebende Selbstkiebefolle verwendet.

Vorzugsweise weist das Schuhelnlagensystem als weiteres Schuheinlagenelement eine Pelotte auf. Die Pelotte ist zwischen dem Rohling und dem Dekkelement anbringbar. Pelotten sind im Rahmen der vorliegenden Erfindung beispielsweise Mittelfußpolster aus Schaumgummi oder Moosgummi mit oder ohne Lederbezug.

Weitere Schuhelnlagenelemente sind Fersenkeile, Fersenkissen und Fersenschalen.

Deckelemente, die im Rahmen der vorliegenden Erfindung eingesetzt werden, bestehen vorzugsweise aus Leder oder synthetischen Materialien. Besonders vorteilhafte Eigenschaften werden durch die Verwendung von Rindsleder erzielt. Das Schuheinlagensystem weist zumindest eine Oberdecke auf, welche auf der zur Fußsohle hin gerichteten Oberfläche des anatomisch geformten Rohlings aufbringbar ist. Zusätzlich zu der Oberdecke weist es eine Unterdecke auf, welche auf die zur Schuhinnensohle hin gerichtete Oberfläche des anatomisch geformten Rohlings aufbringbar ist. Die Oberdecke erstreckt sich über die gesamte Fläche des Fußes. Die Unterdecke erstreckt sich ebenso wie der anatomisch geformte Rohling vorzugsweise über die gesamte Fläche der orthopädischen Einlage einschließlich des Fersenbereiches und des Mittelfußbereiches bis zum Fußballen.

Gemäß einem bevorzugten Ausführungsbeispiel der vorliegenden Erfindung sind die Ober- und Unterdecke so ausgebildet, daß deren Außenkonturen, welche den Außenkonturen des Fußes angepaßt sind, deckungsgleich sind. Die Unterdecke und/oder die Oberdecke werden vorzugsweise etwas größer als der anatomisch geformte Rohling ausgestaltet, so daß im verklebten Zustand ein durch den Rohling nicht gestützter Randbereich ausgebildet ist. Dies hat den Vorteil, daß sich die Schuheinlage besser den inneren Konturen des Schuhes anpaßt und verrutschsicher im Schuh liegt.

Das Klebemittel befindet sich entweder auf dem anatomisch geformten Rohling oder auf dem Dekkelement. Gemäß einem weiteren bevorzugten Ausführungsbeispiel kann das Klebemittel auch auf der Pelotte aufgebracht sein. Das Klebemittel ist auf der gesamten Oberfläche des Bereiches, in welchem die Schuheinlangenelemente später verbunden werden sollen, aufgebracht. Besonders vorteilhaft Ist es, wenn der anatomisch geformte Rohling auf seiner gesamten, zur Fußsohle hin gerichteten Oberfläche und die Unterdekke auf ihrer gesamten, zum Rohling hin gerichteten Oberfläche Klebemittel aufweist.

Gemäß der vorliegenden Erfindung kann das Schuheinlagensystem zum Teil schon vorgefertigt sein, nämlich in der Weise, daß die einzelnen Schuheinlagenelemente schon partiell miteinander verbunden sind. Dies erleichtert den Transport. Es bleiben aber Bereiche von mit dem Überzug bedeckten Klebemittel frei und zwar vorzugsweise dort, wo beispielsweise Pelotten zur individuellen Anpassung an den Fuß eines Patienten des Orthopädie-Schuhmachers anbringbar sind.

Ein anatomisch geformter Rohling, wie es für Schuheinlagen, insbesondere für orthopädische Schuheinlagen, eingesetzt wird, weist eine zur Fußsohle hin gerichtete Oberfläche und eine zur Schuhinnensohle hin gerichtete Oberfläche auf, wobei zumindest eine der Oberflächen ein Klebemittel aufweist, welches zumindest bereichsweise mit einem lösbaren Überzug abgedeckt sein kann. Der Überzug kann bspw. aus Silikonpapier sein und das Klebemittel vollständig überdecken. Silikonpapier weist In Verbindung mit den verwendeten Heißklebemitteln die besten Hafteigenschaften auf und gewährleistet dennoch ein problemloses Transportieren des Rohlings bzw. auch der einzelnen Schuheinlagenelemente. Mittels dem Kleber sind die Schuheinlagenelemente, insbesondere Pelotten und Deckelemente, tragfertig auf der zur Fußsohle hin gerichteten Oberfläche nach Entfernen des Überzugs und Wiedererwelchen des Klebemittels klebend befestigbar.

Das Klebemittel, mit welchem der anatomisch geformte Rohling beschichtet ist, wird in einem Ofen bei Temperaturen In einem Bereich von 75 °C bis 150 °C wieder erweicht, so daß darauf Schuheinlagenelemente, wie beispielsweise Pelotten, aufklebbar sind.

Das Klebemittel behält über einen langen Zeitraum und unter Einwirkung von Körperwärme und Schweiß seine Klebefähigkeit und Elastizität. Durch Behandeln mit Wärme erlaubt das Klebemittel ein wiederholtes Aufbringen und Entfernen der Schuheinlagenelemente zu Anprobezwecken, wobei es nach Entfernen der Schuheinlagenelemente am Rohling verbleibt.

Vorteilhaft wird als wiedererweichbares Klebemittel ein Heißkleber eingesetzt, welcher die oben genannten Vorteile bietet. Es kann aber ebenfalls eine Beschichtung der Schuheinlagenelemente mit einem Kautschukkleber vorgesehen sein.

Das Klebemittel kann sich auf der gesamten zur Fußsohle hin gerichteten Oberfläche und/oder auf der gesamten zur Schuhsohle hin gerichteten Oberfläche befinden. Das Klebemittel kann aber auch nur bereichsweise auf den Oberflächen aufgebracht sein, belspielsweise streifen- oder punktförmig.

Das Klebemittel kann auch auf ein den Rohling umgebendes Vlies aufgebracht werden, was eine noch bessere Haftung sicherstellt. Auch erleichtert der Einsatz eines Vlieses den Herstellungsprozeß für den Rohling, was nachstehend noch näher erläutert wird, und dient gleichfalls zur Verbesserung der optischen Gestaltung des Rohlings.

Der Rohling besteht typischerweise aus einem Kunststoffmaterial, beispielsweise aus einem amorphen Thermoplast, vorzugsweise Polypropylen.

Der Rohling ist anatomisch geformt, d.h. der natürlichen Fußform angepaßt und wird in verschiedenen Schuhgrößen hergestellt. Der Rohling kann sich über die gesamte Fußlänge oder noch besser von der Ferse des Fußes bis zum Beginn des Fußballens, also nur über zwei Drittel der gesamten Fußlänge erstrekken. Die Randbereiche des Rohlings können dabei dünner ausgestaltet sein als die zentralen Bereiche.

Das Klebemittel ist wiedererweichbar und wird mit einer Walzenauftragsmaschine auf die Schuheinlagenelemente aufgebracht. Das wiedererweichbare Klebemittel kann bspw. mit Silikonpapier abgedeckt werden.

Zur Herstellung der tragefertigen Schuheinlage wird ggfs. der Überzug, abgezogen, und anschließend werden die zu verbindenden Schuheinlagenelemente aufeinandergelegt und durch leichtes Andrücken in erhitztem bzw. erweichtem Zustand des Klebemittels miteinander verbunden. Die Schuheinlage ist sofort tragfertig.

Die vorliegende Erfindung weist unter anderem die folgenden Vorteile auf:

Die Schuheinlagenelemente können ohne aufwendige Klebearbeiten zu einer sofort tragfähigen Schuheinlage verbunden werden. Der anatomisch geformte Rohling und des Schuheinlagensystem erlauben eine gegebenenfalls auch mehrmalige idividuelle Anpassung an den Fuß eines Patienten. Pelotten und andere Schuheinlagenelemente können beliebig positioniert werden. Aufgrund dessen, daß kein zusätzliches Klebemittel verwendet werden muß, entstehen beim Zusammenfügen keine gesundheitsgefährdenden Dämpfe. Außerdem kann Klebstoff eingespart werden, da die vorgefertigten Schuheinlagenelemente nur eine sehr dünne Klebstoffschicht bzw. -folie aufweisen. Die vorliegende Erfindung führt zu einer erheblichen Zeitersparnis von bis zu etwa 50% bei der Herstellung von orthopädischen Schuheinlagen. Der Orthopädie-Schuhmacher hat darüber hinaus die Möglichkeit, eigene Schuheinlagenelemente zu dem erfindungsgemäßen Schuheinlagensystem, welches ein Halbfertigprodukt für den Orthopädiebereich darstellt, hinzuzufügen, was eine noch individuellere Anpassung an den Fuß eines Patienten erlaubt, wobei der Orthopädie-Schuhmacher ohne Verwendung weiterer Klebstoffe arbeiten kann.

Weitere Vorteile, Merkmale und Anwendungsmöglichkeiten der Erfindung werden nun anhand von Ausführungsbeispielen unter Bezugnahme auf die beigefügten Zeichnungen detailliert erläutert. Es zeigen:
- Fig. 1: ein Ausführungsbeispiel eines anatomisch geformten Rohlings gemäß der vorliegenden Erfindung mit teilweise abgezogenem Überzug;
- Fig. 2: einen Schnitt entlang der Linie A-A durch den anatomisch geformten Rohling gemäß Fig. 1;
- Fig. 3: ein Schuheinlagensystem gemäß der vorliegenden Erfindung mit teilweise abgezogenem Überzug, bestehend aus a) Oberdecke, b) Rohling mit teilweise abgezogenem Überzug und c) Überzug und Pelotte;
- Fig. 4: eine Draufsicht auf eine orthopädische Schuheinlage, welche aus einem in Fig. 3 dargestellten Schuheinlagensystem hergestellt ist; und
- Fig. 5: einen Schnitt entlang der Linie A-A durch die in Fig. 4 dargestellte Schuheinlage.

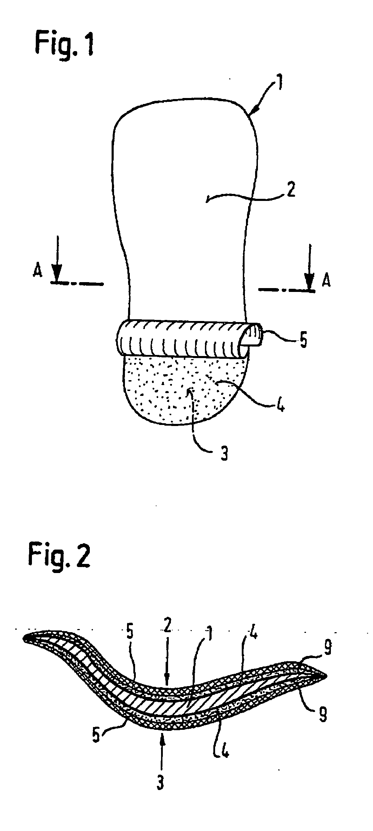

In Fig. 1 ist ein Ausführungsbeispiel eines anatomisch geformten Rohlings 1 für orthopädische Schuheinlagen dargestellt. Die Form das anatomisch geformten Rohlings 1 ist an die natürliche Fußform angepaßt, wobei sich der Rohling 1 nicht über die gesamte Fußfläche erstreckt. Er erstreckt sich von der Ferse des Fußes bis zum Beginn des Fußballens, d.h. über etwa zwei Drittel der gesamten Fußlänge. Der anatomisch geformte Rohling 1 weist eine zur Fußsohle hin gerichtete Oberfläche 2 und eine zur Schuhinnensohle hin gerichtete Oberfläche 3 auf. Auf diesen Oberflächen befindet sich ein Klebemittel 4, welches mit einem lösbaren Überzug 6 abgedeckt Ist. Das Klebemittel 4 und der Überzug 5 befinden sich auf den gesamten zur Fußsohle hin gerichteten Oberflächen 2, 3. Die Außenkonturen des anatomisch geformten Rohlings 1 sind den Außenkonturen des Fußes angepaßt.

Figur 2 zeigt einen Schnitt durch den anatomisch geformten Rohling 1 gemäß Fig. 1 entlang der Linie A-A. Die Randbereiche des anatomisch geformten Rohlings 1 sind dünner ausgestaltet als die zentralen Bereiche. Auf der zur Fußsohle hin gerichteten Oberfläche 2 und der zur Schuhinnensohle hin gerichteten Oberfläche 3 befinden sich Vliese 9, auf die das Klebemittel 4 aufgebracht ist.

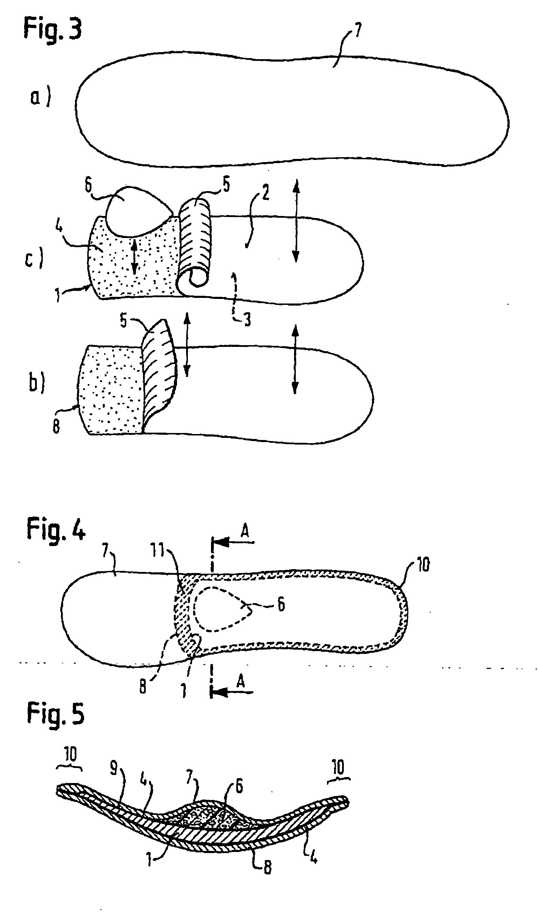

Figur 3 zeigt ein Schuheinlagensystem gemäß der vorliegenden Erfindung. Dieses Schuheinlagensystem weist a) eine Oberdecke 7 und eine nicht dargestellte Unterdecke 8 jeweils aus Leder, b) einen anatomisch geformten Rohling 1 mit abgezogenem Überzug und c) eine Pelotte 6 auf, weiche im Bereich der Gelenke der Fußzehen angeordnet ist. Die Schuheinlagenelemente sind noch nicht miteinander verbunden Der anatomisch geformte Rohling 1 weist auf seiner gesamten, zur Fußsohle hin gerichteten Oberfläche 2 ein Klebemittel 4 auf. Das Klebemittel 4 ist vollständig mit einem lösbaren Überzug 5 abgedeckt. Zwischen dem anatomisch geformten Rohling 1 und dem Klebemittel 4 befindet sich ein Vlies 9 (in dieser Darstellung nicht gezeigt, siehe Fig. 5). Auf der zur Schuhinnensohle hin gerichteten Oberfläche 3 des anatomisch geformten Rohlings 1 befindet sich kein Klebemittel 4. Der Rohling 1 erstreckt sich nicht über die gesamte Fußlänge, sondern über etwa zwei Drittel dergesamten Fußlänge, nämlich von der Ferse des Fußes bis zum Beginn des Fußballens.

Die Pelotte 6 ist ein Kissen aus Moosgummi. Die Oberdecke 7 besteht aus Rindsleder und weist kein Klebemittel 4 auf. Sie erstreckt sich über die gesamte Fußfläche. Die Außenkonturen der Oberdecke 7 entsprechen im wesentlichen den natürlichen Außenkonturen eines menschlichen Fußes. Die Unterdecke 8 besteht aus Leder und weist im wesentlichen ähnliche Außenkonturen auf wie der Rohling 1. Die Unterdecke 8 erstreckt sich also nicht über die gesamte Fußlänge, sondern über einen Bereich von der Ferse bis etwa zum Fußballen. Auf der gesamten, zum Rohling 1 hin gerichteten Oberfläche der Unterdecke 8 befindet sich ein Klebemittel 4, welches vollständig mit einem Überzug 5 abgedeckt ist. Die Unterdecke 8 und die Oberdecke 7 sind größer ausgebildet als der Rohling 1, so daß im verklebten Zustand ein nicht durch den Rohling gestützter. Randbereich 10 ausgebildet ist.

Um die dargestellten Schuheinlagenelemente miteinander zu verbinden, werden die Überzüge 5 von dem Rohling 1 und von der Unterdecke 8 abgezogen und anschließend der Rohling einer Wärmebehandlung unterzogen. Dann wird die Pelotte 6 in der in Fig. 3 dargestellten Weise auf den Rohling 1 an gewünschter Position aufgesetzt und angedrückt. Nachfolgend wird die Oberdecke 7 in der in Fig. 3 dargestellten Anordnung auf den Rohling 1 aufgesetzt und angedrückt. Anschließend wird die Unterdecke 8 auf die zur Schuhinnensohle hin gerichtete Oberfläche 3 des Rohlings 1 aufgebracht. Die in der Weise gefertigte Schuheinlage ist sofort tragbar.

Figur 4 zeigt die in vorstehend beschriebener Welse aus den einzelnen Schuheinlagenelementen zusammengesetzte orthopädische Schuheinlage. Die Figur zeigt, daß die Unterdecke 8 und die Oberdecke 7 größer ausgebildet sind als der Rohling 1, so daß im verklebten Zustand ein nicht durch den Rohling 1 gestützter Randbereich 10 ausgebildet ist. In dem schraffierten Bereich 11 liegen die Oberdecke 7 und die Unterdecke 8 direkt aufeinander und sind dort miteinander verklebt.

In Fig. 5 ist ein Schnitt durch die orthopädische Schuheinlage gemäß Fig. 4 entlang der Linie A-A dargestellt. Die mittlere Lage stellt den anatomisch geformten Rohling 1 dar, auf dessen zur Fußsohle hin gerichteten Oberfläche 2 sich das Vlies 9 und das darauf aufgebrachte Klebemittel 4 befinden. Auf dem Klebemittel 4 sind die Pelotte 6 und die Oberdecke 7 angeklebt. Auf der zur Schuhinnensohle hin gerichteten Oberfläche 3 des anatomisch geformten Rohlings 1 befindet sich das Klebemittel 4, mittels welchem die Unterdecke 8 auf den Rohling aufgebacht wurde. Die Oberdecke 7 und die Unterdecke 8 bilden Randbereiche 10, welche durch den Rohling 1 nicht gestützt sind. In diesen Randberelchen 10 sind die Oberdecke 7 und die Unterdecke 8 direkt miteinander verklebt.

## Patentansprüche

1. Individuell anpassbare, insbesondere orthopädische Schuheinlage, welche mindestens drei verbundene Schuheinlagenelemente, nämlich einen anatomisch geformten Rohling (1), zumindest ein den Rohling (1) vollständig überdeckendes, eine Oberdecke bildendes Deckelement (7) und zumindest eine zwischen Rohling (1) und Oberdecke (7) angeordnete Pelotte (6) umfasst, wobei zumindest eines der Schuheinlagenelemente zumindest im Bereich der Pelotte (6) ein Klebemittel (4) aufweist, wobei das Klebemittel die Schuheinlagenelemente wiederholt thermisch lös- und wiederverklebbar miteinander verbindet,
wobei ferner
die Schuheinlage zusätzlich eine am Rohling (1) schuhsohlenseitig vorgesehene Unterdecke umfasst,
der Rohling (1) sich nicht über die gesamte Fußfläche sondern von der Ferse des Fußes bis zum Beginn des Fußballens, d. h. über etwa 2/3 der gesamten Fußlänge erstreckt ,
die Oberdecke sich über die gesamte Fußfläche erstreckt,
Ober- und Unterdecke (7, 8) größer ausgebildet sind als der Rohling (1),
und wobei im verklebten Zustand ein nicht durch den Rohling (1) gestützter Randbereich (10) ausgebildet ist.

2. Schuheinlage nach Anspruch 1, bei welcher das Klebemittel (4) ein wiedererweichbarer Kleber, insbesondere ein Heißkleber oder ein Kautschukkleber oder eine beidseitig klebende Selbstklebefolie ist.

3. Schuheinlage nach Anspruch 1 oder 2, wobei das Klebemittel auf der gesamten Oberfläche des Bereichs, in welchem die Schuheinlagenelemente verbunden werden sollen, aufgebracht ist.

4. Als Halbfertigprodukt ausgebildetes Schuheinlagensystem für Schuheinlagen nach den Ansprüchen 1 bis 3, insbesondere für den Orthopädiebereich, welches mindestens zwei zu verbindende Schuheinlagenelemente in Form eines anatomisch geformten Rohlings (1) und mindestens eines sich über dessen gesamte Fläche erstreckenden, eine Oberdecke (7) bildenden Deckelements aufweist,
wobei auf dem Rohling (1) und/oder der Oberdecke (7), insbesondere auf der gesamten, jeweils einander zugewandten Oberfläche ein thermisch wiedererweichbares Klebemittel (4) zur wiederholt lösbaren und wiederverklebbaren Verbindung der Schuheinlageelemente aufgebracht ist,
wobei femer
ein weiteres Schuheinlagenelement in Form eines eine Unterdecke bildenden Deckelementes (8) vorgesehen ist, welches im Wesentlichen über seine gesamte, dem Rohling zugewandte Oberfläche mit dem Klebemittel (4) überzogen ist,
der Rohling (1) sich nicht über die gesamte Fußfläche sondern von der Ferse des Fußes bis zum Beginn des Fußballens, d. h. über etwa 2/3 der gesamten Fußlänge erstreckt,
Ober- und Unterdecke (7, 8) größer ausgebildet sind als der Rohling (1),
und wobei im verklebten Zustand ein nicht durch den Rohling (1) gestützter Randbereich (10) ausgebildet ist.

5. Als Halbfertigprodukt ausgebildetes Schuheinlagensystem nach Anspruch 4, welches als weiteres Schuheinlagenelement eine Pelotte (6) aufweist, welche so ausgebildet ist, dass sie zwischen dem Rohling (1) und der Oberdecke (7) anbringbar ist.

6. Schuheinlagensystem nach Anspruch 4 oder 5, bei welchem ein weiteres Schuheinlagenelement in Form eines Fersenkeils oder eines Fersenkissens vorgesehen ist.

## Claims

1. Individually adaptable, in particular orthopaedic shoe insert, which comprises at least three interconnected shoe insert elements, namely an anatomically shaped blank (1), at least one cover element (7) forming a top cover and completely covering the blank (1), and at least one pad (6) arranged between blank (1) and top cover (7), at least one of the shoe insert elements having, at least in the area of the pad (6), an adhesive (4), wherein the adhesive connects the shoe insert elements in such a way that they can be repeatedly detached and reattached by thermal means,
wherein furthermore
the shoe insert additionally comprises a bottom cover provided on the blank (1) on the side facing towards the sole of the shoe,
the blank (1) does not extend over the entire foot area but instead from the heel to the beginning of the ball of the foot, i.e. over about 2/3 of the total foot length,
the top cover extends over the entire foot area, top and bottom cover (7, 8) are made larger than the blank (1), and wherein, in the attached state, an edge region (10) not supported by the blank (1) is formed.

2. Shoe insert according to Claim 1, in which the adhesive (4) is a resoftenable adhesive, in particular a hot-melt adhesive, or a rubber adhesive, or a self-adhesive film which is adhesive on both sides.

3. Shoe insert according to Claim 1 or 2, wherein the adhesive is applied to the entire surface of the region in which the shoe insert elements are to be connected.

4. Shoe insert system designed as a semi-finished product for shoe inserts according to Claims 1 to 3, in particular for the orthopaedic sector, which has at least two shoe insert elements to be connected in the form of an anatomically shaped blank (1) and at least one cover element forming a top cover (7) and extending across the entire surface of the blank (1), wherein, on the blank (1) and/or on the top cover (7), particularly across the entire surface facing the other, an adhesive (4) that can be resoftened by heat is applied in order to permit repeated detachment and reattachment of the shoe insert elements
wherein furthermore
a further shoe insert element is provided in the form of a cover element (8) forming a bottom cover which is coated with the adhesive (4) across substantially the whole of its surface facing towards the blank,
the blank (1) does not extend over the entire foot area but instead from the heel to the beginning of the ball of the foot, i.e. over about 2/3 of the total foot length,
top and bottom cover (7, 8) are made larger than the blank (1),
and wherein, in the attached state, an edge region (10) not supported by the blank (1) is formed.

5. Shoe insert system designed as a semi-finished product according to Claim 4, having, as further shoe insert element, a pad (6) which is designed in such a way that it can be arranged between the blank (1) and the top cover (7).

6. Shoe insert system according to Claim 4 or 5, in which a further shoe insert element in the form of a heel wedge or a heel cushion is provided.

## Revendications

1. Semelle adaptable individuellement, pouvant être orthopédique, qui comprend au moins trois éléments de semelle reliés, à savoir une ébauche (1) de forme anatomique, au moins un élément de recouvrement (7) constituant un revêtement supérieur recouvrant complètement l'ébauche (1) et au moins un rembourrage (6) disposé entre l'ébauche (1) et le revêtement supérieur (7), moyennant quoi au moins un des éléments de la semelle comprend, au moins au niveau du rembourrage (6), un adhésif (4), moyennant quoi l'adhésif relie les éléments de la semelle entre eux de façon à ce qu'ils puissent être détachés et recollés thermiquement plusieurs fois,
moyennant quoi, en outre,
la semelle comprend un revêtement inférieur prévu du côté de la semelle de la chaussure sur l'ébauche (1),
l'ébauche (1) ne s'étend pas sur toute la surface du pied mais seulement du talon jusqu'au début du métatarse, c'est-à-dire sur environ 2/3 de la longueur du pied,
le revêtement supérieur s'étend sur toute la surface du pied,
le revêtement supérieur et le revêtement inférieur (7,8) sont plus importants que l'ébauche (1),
et moyennant quoi, lorsque l'ensemble est collé, un bord (10), non soutenu par l'ébauche (1), est créé.

2. Semelle selon la revendication 1, dans laquelle l'adhésif (4) est une colle qui peut être ramollie, notamment une colle à chaud ou une colle au caoutchouc ou un film adhésif auto-collant à double face.

3. Semelle selon la revendication 1 ou 2, moyennant quoi l'adhésif est appliqué sur toute la surface de la zone dans laquelle les éléments de la semelle doivent être reliés.

4. Système de semelle conçu comme un produit semi-fini, pour des semelles selon les revendications 1 à 3, notamment dans le domaine orthopédique, qui comprend au moins deux éléments de semelle à assembler sous la forme d'une ébauche (1) de forme anatomique et d'au moins un élément de recouvrement qui s'étend sur toute la surface de l'ébauche et qui constitue un revêtement supérieur (7), moyennant quoi, sur l'ébauche (1) et/ou sur le revêtement supérieur (7), plus particulièrement sur les surfaces orientées l'une vers l'autre, un adhésif pouvant être ramolli thermiquement (4) est appliqué pour l'assemblage des éléments de la semelle pouvant être détachés et recollés plusieurs fois,
moyennant quoi, en outre,
un autre élément de semelle, sous la forme d'un élément de revêtement (8), constituant un revêtement inférieur, est prévu qui est recouvert d'adhésif (4) sur toute sa surface orientée vers l'ébauche,
l'ébauche (1) ne s'étend pas sur toute la surface du pied mais seulement du talon jusqu'au début du métatarse, c'est-à-dire sur environ 2/3 de la longueur du pied,
le revêtement supérieur et le revêtement inférieur (7,8) sont plus importants que l'ébauche (1),
et moyennant quoi, lorsque l'ensemble est collé, un bord (10), non soutenu par l'ébauche (1), est créé.

5. Système de semelle conçu comme un produit semi-fini selon la revendication 4, qui comprend, en tant qu'élément supplémentaire de la semelle, un rembourrage (6) conçu de telle sorte qu'il puisse être disposé entre l'ébauche (1) et le revêtement supérieur (7).

6. Système de semelle selon la revendication 4 ou 5, dans lequel un élément supplémentaire de la semelle est prévu sous la forme d'une cale de talon ou d'un coussin de talon.
